# EUROPEAN PATENT SPECIFICATION

(11) **EP 0 483 888 B1**
(45) Date of publication and mention of the grant of the patent: **01.03.1995**
(21) Application number: 91202042.7
(22) Date of filing: 09.08.1991
(51) Int. Cl.: A23L 1/23, A23L 1/226, A23L 1/22, A23C 9/123

(54) **Process for preparing an aroma product containing alpha-acetolactic acid**
Verfahren zur Zubereitung eines Aroma-Produkts, das Alpha-Acetomilchsäure enthält
Procédé pour la préparation d'un produit d'arôme contenant de l'acide alpha-acétolactique

(30) Priority: 13.08.1990 EP 90202198; 11.09.1990 GB 9019810
(43) Date of publication of application: 06.05.1992
(73) Proprietor: UNILEVER N.V., 3013 AL Rotterdam (NL); UNILEVER PLC, London EC4P 4BQ (GB)
(72) Inventor: Verhue, Walter Maurits M., UNILEVER Research, NL-3133 AT Vlaardingen (NL); Tjan, Sing Boen, UNILEVER Research, NL-3133 AT Vlaardingen (NL); Verrips, Cornelis Theodorus, UNILEVER Research, NL-3133 AT Vlaardingen (NL); van Schie, Bartholomeus Josef, UNILEVER Research, NL-3133 AT Vlaardingen (NL)
(74) Representative: Sikken, Antonius H. J. M.

(56) References cited:
- EP-A- 0 073 629
- EP-A- 0 247 646
- US-A- 4 670 267
- PATENT ABSTRACTS OF JAPAN vol. 12, no. 96 (C-484), 4
- December 1986; & JP - A - 62228282 (KIRIN BREWERY CO. LTD.) 07.10.1987

## Description

### BACKGROUND OF THE INVENTION

The present invention relates to a process for preparing an aroma product containing a high concentration of α-acetolactic acid by subjecting at least one food product constituent to lactic acid bacterial matter.

Diacetyl is an important flavour for dairy products like cottage cheese, butter and buttermilk. In yoghurt small quantities of diacetyl are also required to obtain a well balanced flavour. Diacetyl is an important flavour also for margarines and low fat spreads in general and for bakery products. While preparing the latter, it is advantageous to include the diacetyl precursor α-acetolactic acid instead of diacetyl itself, since diacetyl is formed from the precursor during the baking or frying process. The addition of α-acetolactic acid instead of diacetyl to foodstuffs has the additional advantage that α-acetolactic acid is much less volatile than diacetyl and thus the aromatizing effect can be maintained for a longer time provided that the α-acetolactic acid can be kept stable.

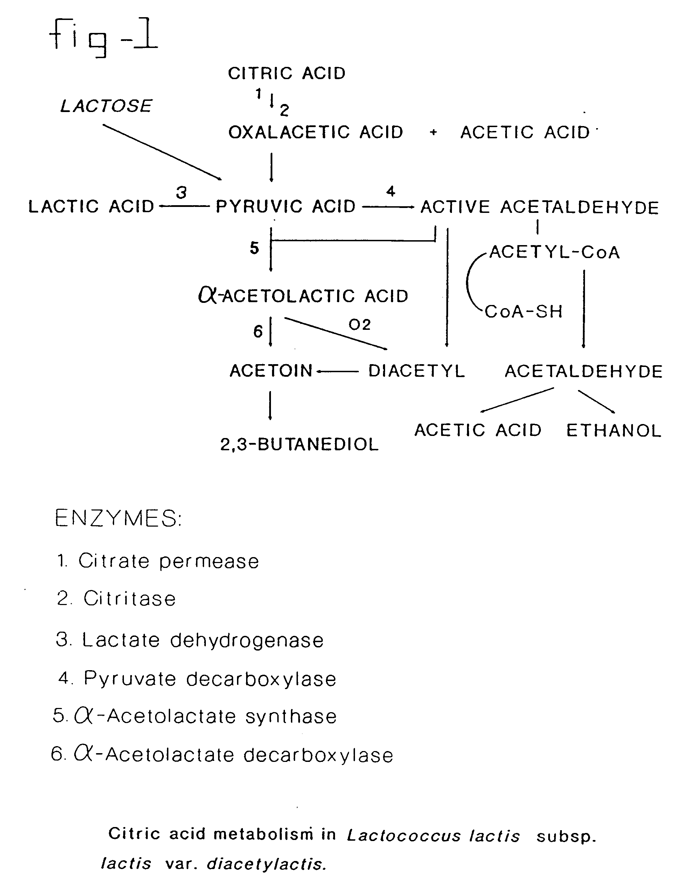

The dairy industry uses mainly two species of lactic acid bacteria to obtain diacetyl in their products. These are *Leuconostoc cremoris* and especially *Lactococcus lactis* subspecies *lactis* biovar *diacetilactis* (hereinafter also abbreviated as *L. diacetilactis*). The biosynthesis of diacetyl by the latter species has been studied in detail. It is generally agreed that citric acid, which is present in milk at some 0.15% (w/v), is the milk component from which diacetyl is produced. Pyruvic acid however can serve as the precursor as well (T.M. Cogan, J. Dairy Research 51 (1984) 597). From there on, opinions diverge as to the exact way in which diacetyl is produced. Two pathways have been described for the production of diacetyl; these pathways are illustrated in Fig. 1. The first pathway involves the combination of acetaldehyde-TPP and acetyl-CoA directly into diacetyl. The second pathway concerns the formation of the diacetyl precursor α-acetolactic acid by combination of pyruvic acid and acetaldehyde-TPP. The diacetyl precursor can afterwards be converted into diacetyl by oxidative decarboxylation, which can be achieved by physical means such as heating or stirring in the presence of air or oxygen, preferably at low pH.

A particular culture is described in the literature (H.A. Veringa *et al*., Milchwissenschaft 31 (1976) 658), which is able to produce a relatively high concentration of diacetyl/α-acetolactic acid. We have investigated this culture (containing several strains) and selected strains and we determined the enzyme activities of those strains, which are related to the production of α-acetolactic acid. We found that the level of the enzyme α-acetolactate decarboxylase in an isolated *L. diacetilactis* strain from the culture described by Veringa *et al*., which is responsible for the decarboxylation of α-acetolactic acid to acetoin, is lower than in other strains of *L. diacetilactis*, thereby building up higher concentrations of α-acetolactic acid. Most probably, this holds for other productive strains as well. A strain capable of producing high amounts of α-acetolactic acid, isolated from the commercially available Flora Danica IBA culture from "Christian Hansen Laboratorium", Denmark and given the name SD803 was incorporated into a culture, that was used to develop a fermentation/spray-drying process wherein the spray-dried powder contains α-acetolactic acid. One of the other strains was obtained from McKay and described in L.L. McKay & K.A. Baldwin, J. Dairy Sci. 57 (1974) 181, which is a mutant having lost its property to convert pyruvic acid to lactic acid, which conversion and subsequent secretion of the lactic acid is energetically essential for the organism. Based on earlier work we had expected that this mutant when grown on a suitable carbon source would produce a higher amount of α-acetolactic acid, because the alternative route of converting pyruvic acid is blocked. Indeed we found an increased level of intracellular pyruvic acid. However, this did not result in an increased production of α-acetolactic acid. Instead an elevated level of ethanol was found.

An aroma composition containing α-acetolactic acid and diacetyl is described in European Patent Application EP-A-0247646. The composition can contain 0.05% or more of α-acetolactic acid. It can be prepared by fermenting a pasteurized or sterilized milk product with a selected culture containing *Streptococcus diacetilactis*, which is now known as *Lactococcus lactis* subspecies *lactis* biovar *diacetilactis*, taking measures to decrease the conversion of α-acetolactic acid into diacetyl; such measures are pH regulation, use of a milk product as a fermentation medium and addition of citric, oxaloacetic and/or pyruvic acid. That document thus describes a process for preparing a dry aroma composition containing α-acetolactic acid, but the content of α-acetolactic acid of the product is still rather low. As a consequence, the use of such an aroma composition may require the addition of such a large quantity of the dry aroma component that its use becomes too expensive for some products or that the carrier material present in that composition interferes with the functionality of such products.

### SUMMARY OF THE INVENTION

The present invention provides a process for preparing an aroma product containing a high concentration of α-acetolactic acid by subjecting at least one food product constituent to lactic acid bacterial matter, which comprises subjecting a sufficient amount of said at least one food product constituent to lactic acid bacteria which are no longer in their logarithmic growth phase, or to a cell extract or a cell lysate of the lactic acid bacteria.
In a preferred embodiment the lactic acid bacteria have been separated from their culture medium.
According to another aspect of the invention the food product constituent is selected from the group consisting of citric acid, lactic acid and pyruvic acid, and mixtures thereof. If pyruvic acid is used, the latter is preferably obtained from a culture of at least one bacterium of the genus *Propionibacterium*, preferably of the species *Propionibacterium freudenreichii*. Preferably a medium is used, which is obtainable by culturing at least one bacterium of the genus *Propionibacterium* in an anaerobic nutrient medium to produce biomass and subsequently continuing to culture aerobically in a medium containing lactic acid until a pyruvic acid level of at least 3 g/l, preferably at least 5 g/l, and more preferably at least 10 g/l, is reached.
According to still another aspect of the invention the lactic acid bacteria are selected from the group consisting of the genera *Lactococcus*, *Streptococcus*, *Lactobacillus*, *Leuconostoc* and *Pediococcus*, preferably of the species *Lactococcus lactis*, more preferably of *Lactococcus lactis* subspecies *lactis*. Particularly preferred is the use of a bacterium of *Lactococcus lactis* subspecies *lactis* biovar *diacetilactis*, especially *Lactococcus lactis* sub-species *lactis* biovar *diacetilactis* strain SD803.
A further aspect of the invention is an aroma product containing α-acetolactic acid in addition to other food fermentation products, which comprises at least 0.1 wt.%, preferably at least 0.2 wt.%, more preferably at least 0.4 wt.%, of α-acetolactic acid. Preferably the aroma product is dry and comprises at least 0.2 wt.%, preferably at least 1 wt.%, more preferably at least 2 wt.% of α-acetolactic acid.
Another aspect of the invention relates to aromatized food material, which comprises such aroma product or an aroma product obtained according to a process according to the invention as described above and to a process for preparing such aromatized food material wherein the aroma product is mixed with a pre-dried food material, for example whey powder, skim milk powder, wheat flour or a polysaccharide.

### DETAILED DESCRIPTION OF THE INVENTION

According to the present invention a new and superior process is provided for producing the diacetyl precursor α-acetolactic acid, in high concentrations and in nearly pure form.

The process for preparing an aroma product according to the invention comprises subjecting a food product constituent to lactic acid bacteria which are no longer in their logarithmic growth phase.

A particular advantage of the process according to the invention is that the production of α-acetolactic acid is not limited to certain selected strains of *L*. *diacetilactis*, but that this product can be obtained by the species in general; furthermore, other lactic acid bacteria, e.g. *Lactococcus lactis* subspecies *lactis*, *Lactococcus lactis* subspecies *cremoris*, *Lactobacillus casei*, *Leuconostoc cremoris* etc. can be used to obtain the same product.

It has been found that the treatment of a food product constituent with bacteria which are no longer in their logarithmic growth phase results in a higher content of α-acetolactic acid than a treatment of an actively growing culture of these bacteria. Lactic acid bacteria which are no longer in their logarithmic growth phase, i.e. which have reached the stationary phase, are bacteria which are in a stage wherein the conversion of pyruvic acid to lactic acid and the subsequent efflux of lactic acid from the cytoplasm into the medium is no longer sufficient to generate enough energy for cell growth and also no longer sufficient for recycling NADH. The bacteria to be used in the process of the invention are in the stationary phase after having been precultured in a suitable medium to obtain a desirable biomass.

Preferably, the lactic acid bacteria have been separated from their culture medium. Separation of the bacteria or bacterial matter from the culture medium can be effected amongst other methods by centrifugation or (membrane) filtration, followed by washing the bacteria with an aqueous liquid. According to another preferred embodiment of the present process, cell lysates or cell-free extracts rather than whole live cells are used to convert the substrate into product. Cell lysates are understood to be the product obtained after disruption of the bacterial cells by any appropriate physical means, e.g. homogenization. A cell-free extract is obtained by removing all particulate matter from a lysate, e.g. by centrifugation, with the purpose to obtain soluble cell matter only.

The use of cell lysates or cell-free extracts instead of whole cells is preferred for two reasons: 1) the rate of conversion of the substrate into the product is much higher, most probably because the permeability of the cell membrane for the relevant substrates is rate limiting; 2) the conversion of pyruvic acid into α-acetolactic acid is nearly quantitative, which cannot be achieved with live cells.

A food product constituent is to be understood to include any constituent of food materials, e.g. skimmed milk, curd or whey, or produced from such materials by the action of lactic acid bacteria. Other food products or derivatives thereof, e.g. meat or plant extracts, can also be used provided that they contain one or more of the substrates required for the production of α-acetolactic acid. The food product constituent is preferably derived from a milk product and comprises components occurring in the metabolic scheme as depicted in Fig. 1. These constituents include citric acid, lactic acid and pyruvic acid. Most preferably, the food product constituent is pyruvic acid, optionally together with lactic acid and/or citric acid. In this art the terms 'pyruvic acid' and 'pyruvate' are usually exchanged. Which compound is present in a specific product depends on the pH. Therefore, the organic acids mentioned in the present application, including pyruvic acid, lactic acid, citric acid and α-acetolactic acid, are to be understood as to include both the free acids and their nutritionally acceptable salts, e.g. sodium and potassium salts. When the term pyruvate etc. is used it does not necessarily mean the salt, but can also stand for the corresponding acid.

It has further been found that pyruvic acid, a preferred substrate for the α-acetolactic acid producing process according to the present invention, can be prepared in an improved fermentation process using a bacterium of the genus *Propionibacterium*, (for a review of the genus *Propionibacterium*, see Bergey's Manual of Systematic Bacteriology **1986**, page 1346). Preferably the organism is of the species *Propionibacterium freudenreichii* and especially of the subspecies *shermani*.

A pyruvic acid solution can be prepared by culturing at least one member of the genus *Propionibacterium* under anaerobic conditions in a nutrient medium, e.g. whey or enriched whey containing lactose as the substrate, to produce sufficient biomass, preferably one having an optical density at 660 nm of at least 5, and continuing to culture aerobically with lactic acid as substrate until a sufficiently high pyruvic acid level is reached. The aerobic fermentation can also be carried out under cell-recycle conditions with continuous removal of spent medium and replenishment with fresh medium. This process resulted in a medium suitable for use in a process of producing α-acetolactic acid according to the invention, which medium contains at least 3 g/l, preferably at least 5 g/l, more preferably at least 10 g/l of pyruvic acid.

In principle, the process of producing α-acetolactic acid according to the invention can be carried out using any lactic acid bacteria or lysate or cell-free extract thereof. The lactic acid bacteria can be selected from the genera *Lactococcus, Streptococcus, Lactobacillus, Leuconostoc and Pediococcus*. Preferably, a species of the genus *Lactococcus or Leuconostoc* is used. More preferably, the selected species is a *Lactococcus* species, in particular the species *Lactococcus lactis*, of which *Lactococcus lactis* subspecies *lactis* is preferably used, more preferably the biovar *diacetilactis* thereof.

*Lactococcus lactis* subspecies *lactis* biovar *diacetilactis* is to be understood to comprise all taxonomic denominations, e.g. *Lactococcus lactis* subspecies *diacetilactis, Lactococcus diacetilactis*, and *Streptococcus diacetilactis*.

Particularly useful results are obtained by using the *Lactococcus lactis* subspecies *lactis* biovar *diacetilactis* strain SD803.

The process according to the invention can be carried out using a virtually homogeneous mixture of food product constituents, lactic acid bacterial matter and medium. However, it is sometimes preferred, especially for a continuous process mode, to use an immobilized bacterial matter. The cell-free extract or lysate of the lactic acid bacteria can for example be contacted on a solid carrier. This means that the bacterial matter is immobilized onto or enclosed into a suitable matrix, e.g. alginate, or enclosed in an apparatus, e.g. a hollow fibre reactor. Another useful embodiment is the use of a packed bed of the bacterial lysate, whereas it may also be advantageous to use a double membrane system in which the membranes are permeable to both the substrate and product molecules, e.g. pyruvic acid, citric acid and α-acetolactic acid, and impermeable to the bacterial fragments.

The process according to the invention results in a mixture containing a high level of α-acetolactic acid. The α-acetolactic acid content is substantially higher than the concentration which can be achieved using the process according to EP-A-0247646: in the fermentation product the highest concentration disclosed is 575 mg/kg (see Example VIII) whereas in the dried product the highest concentration disclosed is 1280 mg/kg (see Example III). The α-acetolactic acid content in a fermentation product obtained in a process according to the invention can be at least 0.1 wt.%, preferably at least 0.2 wt.% and more preferably at least 0.4 wt.% before drying. Preferably, the product of the process according to the invention is subsequently dried, for example by freeze-drying or spray-drying, preferably using moderate temperatures. Such a dried product can contain at least 0.2 wt.%, preferably at least 1 wt.% and more preferably at least 2 wt.%, of α-acetolactic acid.

The invention also relates to aroma products which, in addition to other food fermentation products, contain at least 0.4% by weight of α-acetolactic acid and in particular at least 2% by weight of α-acetolactic acid after drying.

The product α-acetolactic acid, which is obtained in nearly pure form by applying the process of this invention, can be used either as a solution or as a dried powder or it can be sprayed on other dry ingredients under such conditions that the loss of α-acetolactic acid is minimized, to flavour all types of products.

The invention encompasses aromatized food materials. Such aromatized food materials are preferably prepared by adding the α-acetolactic acid-containing aroma product to a pre-dried food material, e.g. whey powder, skim milk powder, wheat flour or polysaccharides. The α-acetolactic acid contained in such aromatized food material is surprisingly stable and these materials can be further processed into foodstuffs wherein diacetyl constitutes a desired flavour. Foodstuffs which can be aromatized according to the invention comprise a wide range of food materials e.g. butter, butter substitutes and other spreads, baking materials e.g. cake mixes, flour, bakery fats etc. Preferably, the aroma product containing α-acetolactic acid is used at such a rate, that the aromatized food material has a concentration of α-acetolactic acid between about 1 and about 100 mg/kg.

The invention is illustrated by the following examples.

### EXAMPLE 1

*L. diacetilactis* strain SD803 was cultivated in M17 broth which contained 1% (w/v) of lactose and which was supplemented with 0.2% (w/v) of citric acid. After cultivating for 18 hours at 20°C the cells were harvested by centrifugation at 7000 rpm for 15 minutes. Subsequently the cells were washed once with physiological salt solution and taken up in phosphate buffer (0.002 mole/l) at pH 7.0 until an optical density at 610 nm of 12.0. The cells were lysed by homogenizing 5 g of cell suspension ultrasonically with 3 g of acid-washed glass beads (<150 microns) for 6 x 1 minute with intermittent cooling for 0.5 minute. Either the complete lysate or the lysate cleared by centrifugation was used to convert 3-¹³C-pyruvate, which was contained in a reaction volume of 1 ml.
The following reaction mixture was used:

| | |
|---|---|
| Thiamine pyrophosphate (300 »g/ml) | 300 »l (= 0.25 mmole/l) |
| Phosphate buffer (0.2 mole/l, pH 6.5) | 300 »l (= 0.06 mole/l) |
| MgSO₄ (0.15 mmole/l) | 10 »l |
| Extract | 150 »l |
| D₂O | 100 »l |
| 3-¹³C-pyruvate (100 »g/ml) | 40 »l (= 0.036 mole/l) |
| H₂O | 110 »l |
| | 1̅0̅0̅0̅ »̅l̅ |

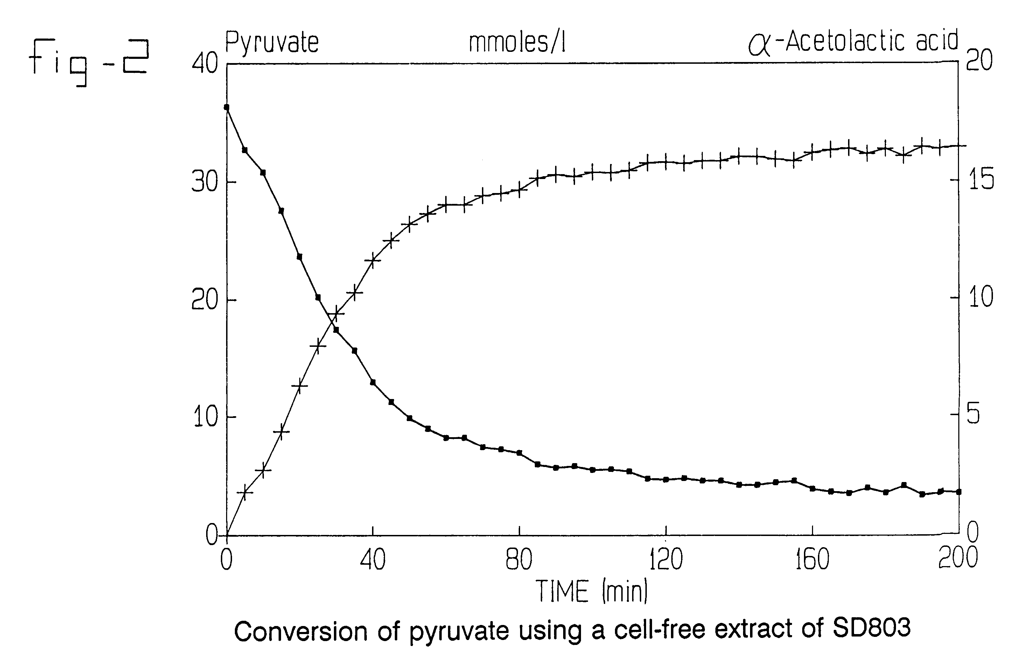

The 3-¹³C-pyruvate was used to facilitate the quantification of the reaction products (by ¹³C-NMR) and has no special property in the conversion process. The protein content of either lysate or extract was standardized at 0.9 mg protein/ml in the experiment.
As shown in Fig. 2, about 30 »mole of pyruvate (83% of the amount of pyruvate added) was used within 80 minutes and converted exclusively to α-acetolactic acid (15 »mole). This is equivalent to 0.21 mmole per gram protein per minute. Until 3.5 hours of incubation, no acetoin was found.
The experiment was repeated using strains SDC6, NMU71 and DRC3 respectively, with the following results:
Strain SDC6: until 50 min. of incubation all pyruvate converted was found as α-acetolactic acid. Thereafter the α-acetolactic acid decreased and acetoin increased.
Strains NMU71 and DRC3 : as with SDC6, decrease of α-acetolactic acid and increase of acetoin slightly faster.
For comparison a fermentation process (using whole cells instead of a lysate, the strain SD803 and an identical concentration of pyruvate) yielded about 7 »mole of α-acetolactic acid after 2 hours. This corresponds to 0.06 mmole per gram protein per minute, the balance being lactic acid.

### EXAMPLE 2

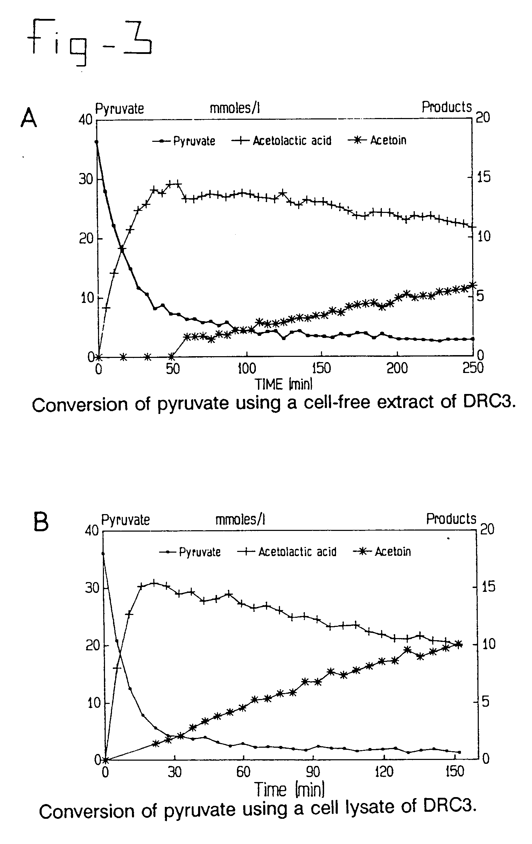

The experiment as reported in Example 1 was repeated, now using *L. diacetilactis* strain DRC3 instead of SD803. Both the cell lysate and the cell-free extract were used. As shown in Figs. 3A and 3B, about 29 »mole of pyruvate were converted to α-acetolactic acid within 60 minutes using the extract and about 30 »mole within 20 minutes using the lysate. At these times, no other product was formed yet. Upon continuation of the experiment, acetoin formation started, at the expense of α-acetolactic acid. In a comparative fermentation process and using the same strain and identical concentration of pyruvate, no α-acetolactic acid was formed at all. Only lactic acid and acetoin could be detected as reaction products.

### EXAMPLE 3

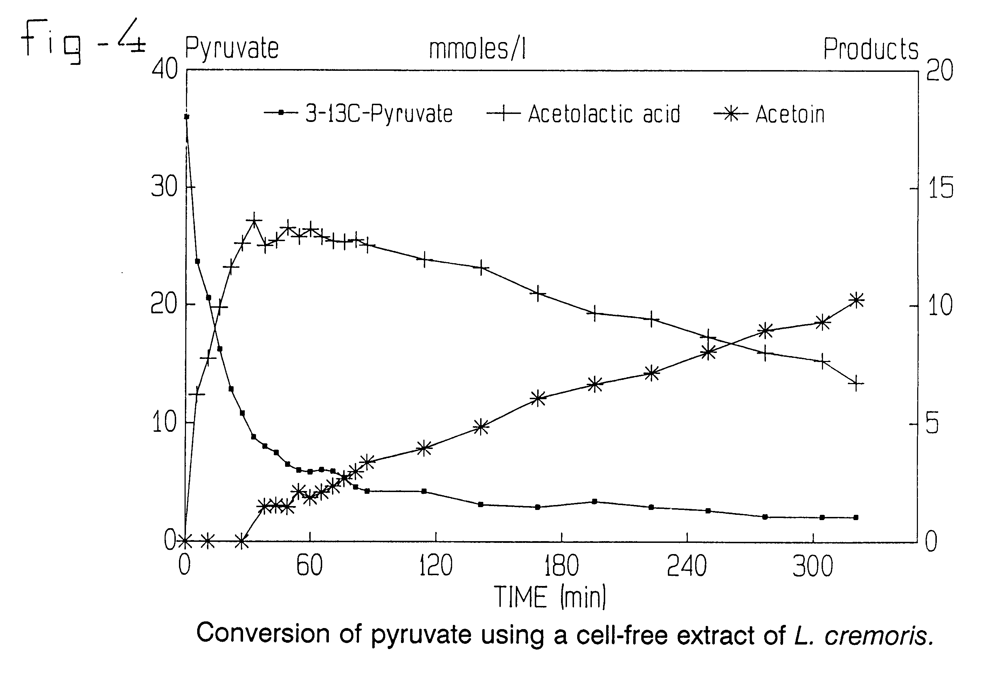

The experiment as reported in Example 1 was repeated, now using a strain of *L. lactis* subspecies *cremoris* instead of *L. diacetilactis*.
As shown in Fig. 4, about 27 »moles of pyruvate were converted mainly to α-acetolactic acid within 30 minutes. At that time, acetoin formation started, at the expense of α-acetolactic acid.
In a comparative fermentation process and using the same strain and an identical concentration of pyruvate, no α-acetolactic acid was formed at all. Only lactic acid could be detected as a reaction product.

### EXAMPLE 4

*L. diacetilactis* strain SD803 was cultivated in M17 broth containing 1% (w/v) of lactose and 0.2% (w/v) of citric acid for 7 hours at 30°C. Then 3-¹³C-pyruvate was added up to a concentration of 45 mmole/l and cultivation was continued for another 3 hours. Using ¹³C-NMR, a level of 7 mmole/l of α-acetolactic acid was found, i.e. a yield of about 15%.

### EXAMPLE 5

*L. diacetilactis* strain SD803 was cultivated in M17 broth which contained 1% w/v of lactose and 0.2% (w/v) of citric acid, for 7 hours at 30°C. Then 2,4-¹³C-citrate was added up to a concentration of 20 mmole/l and cultivation was continued for another 3 hours. Using ¹³C-NMR, a level of 5 mmole/l of α-acetolactic acid was found, i.e. a yield of about 50%.
Similar results were obtained when using milk instead of M17 broth.

### EXAMPLE 6

### 1. Preparation of cell homogenate.

7.5 litres of a culture of *L. diacetilactis* were added to 150 litres of medium which contained 3 kg whey powder, 3 kg lactose, 1.1 kg yeast extract and 0.15 kg sodium citrate. The pH of the medium was 6.3. The culture was grown at 30°C during 18 hours. The cell count was then 1.5 x 10⁹ per ml. The biomass was concentrated to 5 litres using a Westfalia type SA-1 disk centrifuge. The cell count was then 4 x 10¹⁰ per ml.
The biomass was disrupted by 4 passes through an APV Gaulin 30 CD homogenizer at 800 bar. The viable count after this treatment was 8 x 10⁹ per ml, which represents 20% of the viable count before homogenization. The homogenate was stored at -20°C until use.

### 2. Preparation of pyruvate.

100 litres of medium containing 3 kg whey powder, 2 kg sodium lactate, 1 kg yeast extract, 0.5 g MnSO₄, 0.66 kg KH₂PO₄ and 0.31 kg K₂HPO₄ at pH 6.4 were inoculated with 1 litre of a culture of *Propionibacterium freudenreichii* subspecies *shermani*. Growth proceeded anaerobically (nitrogen in head space of fermenter) at 30°C during 48 hours. At this time an OD₆₁₀ of 8.0 was reached and the culture was converted to an aerobic phase by sparging air and maintaining a dissolved oxygen tension of at least 20% of the saturation level of oxygen in the liquid. At the same time a continuous feed of concentrated sodium lactate solution at a rate of 5 g per l of culture medium per hour was applied. This phase lasted 20 hours (100 g of lactate added per litre).
The biomass was centrifuged off using a disk centrifuge. The pyruvate concentration in the supernatant was 22 g/litre, as determined by HPLC. It was stored at -20°C until use.

### 3. Preparation of α-acetolactic acid.

To 40 l of pyruvate solution was added: 4 l of cell homogenate, 108.4 g of MgSO₄.7H₂O (final concentration 10 mmole/l) and 4.1 g of Cocarboxylase (thiamine pyrophosphate, final concentration 0.2 mmole/l). The reaction proceeded during 3 hours at 30°C. The reaction mixture was cooled to 5°C and analyzed.

### 4. Analysis.

Analysis of the components of the reaction mixture was carried out using two methods: HPLC and ¹³C-NMR. The following were determined by HPLC: pyruvic acid, lactic acid, acetic acid, propionic acid. Acetoin and α-acetolactic acid were determined by ¹³C-NMR. It has to be understood that using ¹³C-NMR one determines the molar proportions of the components, not the absolute concentrations themselves. In order to obtain absolute concentrations, reference must be made to an absolute concentration, determined by HPLC, of a reference compound. The concentration of propionic acid was used as the reference.
The following results were obtained:

| | mmole/l | g/l |
|---|---|---|
| Pyruvic acid | 0 | 0 |
| Lactic acid | 207 | 18.6 |
| Acetic acid | 45 | 2.7 |
| Propionic acid | 107 | 7.9 |
| Acetoin | 41.8 | 3.7 |
| α-Acetolactic acid | 40.5 | 5.3 |

Since the starting concentration of pyruvic acid was 250 mmole/l (22 g/l) and two moles of pyruvic acid are needed to make one mole of α-acetolactic acid, the yield of α-acetolactic acid was 32.4 %.

### EXAMPLE 7: Preparation pyruvic acid suitable as a substrate for the production of α-acetolactic acid.

The following example was performed both with a strain of *Propionibacterium freudenreichii* (ATCC 9617) and with a strain of *Propionibacterium freudenreichii* subspecies *shermani (VBCPr 02-01)*.
An inoculum was prepared from a stab, by culturing the microorganism in a 250 ml stoppered flask without agitation for four days at 30°C (waterbath) under anaerobic conditions. The culture medium for this growth phase was MRS broth (250 ml) ex. Merck, Darmstadt FRG.
Bulk culture was initiated by inoculation of four day inoculum into a 15 litre fermenter (ex. Applicon, Schiedam, NL) with an operating volume of 10 litre, stirring at 300 rpm, under anaerobic conditions. During this anaerobic phase the culture medium was sparged with nitrogen at 0.5 l/min. The culture medium was as follows:

| | |
|---|---|
| MnSO₄ | 0.05 g/l |
| MgSO₄ | 0.1 g/l |
| KH₂PO₄ | 2.0 g/l |
| Yeast extract (PTK) | 10 g/l (ex. Ohly, FRG) |
| Trypticase Peptone | 10 g/l (ex. Hy-cas, USA) |
| Glucose | 30 g/l (ex. Avebe, Veendam, NL) |

The pH of the culture medium was lowered to 4.0 prior to sterilisation (obtained by the addition of phosphoric acid, 85% ex. Merck, Darmstadt. FRG), so as to avoid any Maillard reaction during sterilisation.
After sterilisation of the culture medium 1.0 ml/l of 'Egli' micro nutrients and 1.5 ml/l of 'MEM' vitamin solution (ex. Sigma) were added. The composition of the 'Egli' solution was as follows:
Egli micro-nutrient solution:

| | | | |
|---|---|---|---|
| Distilled water | 1000 ml | Ammonium Chloride | 7.63 g |
| KH₂PO₄ | 2.81 g | MgSO₄.7H₂O | 0.59 g |

and 10 ml of a micronutrient solution consisting of an aqueous solution containing

| | | | |
|---|---|---|---|
| CaCl₂ | 5.50 g/l | FeSO₄ | 3.75 g/l |
| MnSO₄ | 1.40 g/l | ZnSO₄ | 2.20 g/l |
| CuSO₄ | 0.40 g/l | CoCL₂ | 0.45 g/l |
| NaMoO₄ | 0.26 g/l | H₃BO₄ | 0.40 g/l |
| KI | 0.26 g/l | Na-EDTA | 30.0 g/l. |

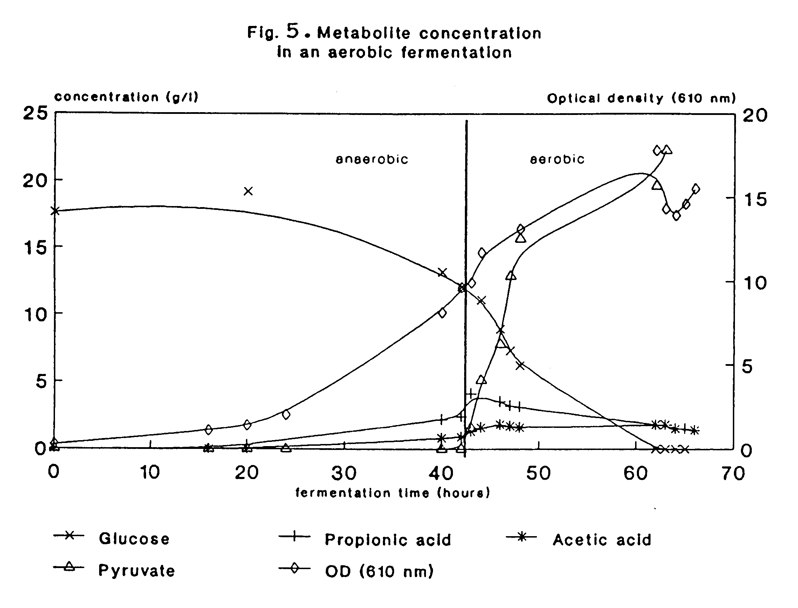

The culture was batch grown for 42 hours at 30°C in the fermenter under anaerobic conditions. The pH was held at 6.5 by automated addition of sodium hydroxide solution (4 mole/l).
Analysis revealed that during anaerobic fermentation the major metabolic products were propionic acid and acetic acid. A small quantity of succinic acid was produced.
After 42 hours duplicate experiments were performed with and without a change-over to aerobic conditions.
Under anaerobic conditions the nitrogen sparging was continued. For aerobic conditions, the nitrogen sparging was discontinued. Aerobic conditions were maintained by sparging with air (0.5-2.0 l/min) and the stirring rate was increased to 700-1000 rpm.
At three times during the subsequent fermentation 300 ml of sodium lactate solution was added to the culture medium (sodium lactate 60%, ex. CCA biochem, Gorinchem, NL).
During aerobic fermentation the main metabolic product was pyruvic acid. Little or no propionic acid or acetic acid were produced. Medium component levels for the ATCC strain are shown in Figure 5 and given in the following TABLE.

**TABLE**

| Results of the analysed samples of *P*. *freudenreichii* Aerobic and anaerobic fermentation (see Figure 5). | | | | | | | |
|---|---|---|---|---|---|---|---|
| Time (h) | anaerobic | | | aerobic | | | |
| | acetic acid (g/l) | propionic acid (g/l) | succinic acid (g/l) | acetic acid (g/l) | propionic acid (g/l) | pyruvic acid (g/l) | succinic acid (g/l) |
| 42 | 1.1 | 3.2 | 0.1 | 1.2 | 3.6 | 0.0 | 0.0 |
| 43 | 1.2 | 3.6 | 0.6 | 1.3 | 4.1 | 1.6 | 0.0 |
| 44 | 1.4 | 4.5 | 1.1 | 1.6 | 4.1 | 5.1 | 0.0 |
| 46 | 1.7 | 5.7 | 1.6 | 1.8 | 3.5 | 7.8 | 0.0 |
| 47 | 1.7 | 5.9 | 1.7 | 1.7 | 3.2 | 12.9 | 0.0 |
| 48 | 1.7 | 6.1 | 2.0 | 1.7 | 3.1 | 15.7 | 0.0 |
| 62 | 2.6 | 14.4 | 5.3 | 1.8 | 1.8 | 19.6 | 0.0 |
| 63 | 2.6 | 14.8 | 5.7 | 1.7 | 1.8 | 22.3 | 0.0 |
| 64 | 2.7 | 15.6 | 6.7 | 1.7 | 1.7 | - | 0.0 |
| 65 | 2.8 | 16.3 | 6.8 | 1.7 | 1.7 | - | 0.0 |

Pyruvic acid concentration was determined by HPLC (Bio-Rad, model 1755 with HPX 87 H Animex column). Samples for HPLC were pressed through a millipore microfilter (0.45 »m) to remove all biomass and the filtrate was diluted 20 times with HPLC-eluent (0.005 Molar H₂SO₄). Pyruvic acid has a retention time of 9.8 minutes, making it distinguishable from glucose (8.9 minutes) and succinic acid (11.7). The other components were determined by similar methods.
Both strains produced a final level of pyruvic acid of 24 g/l under aerobic conditions.
The above mentioned example was repeated with a fed batch addition of substrate and with a culture of *P*. *freudenreichii* subspecies *shermani*. The final level of pyruvic acid achieved was 20 g/l. It is believed that the fed batch addition resulted in a lower pyruvic acid concentration due to too high an addition rate of lactate.

### Legends to the Figures

Figure 1 describes two alternative schemes for the production of diacetyl.
Figure 2 gives the conversion of pyruvic acid using a cell-free extract.
   - **- - -**: gives the pyruvic acid concentration
   - + + +: gives the α-acetolactic acid concentration
Figure 3A gives the conversion of pyruvic acid using a cell-free extract of *L. diacetilactis* strain DRC3.
Figure 3B gives the conversion of pyruvic acid using a cell lysate of *L. diacetilactis* strain DRC3.
Figure 4 gives the conversion of pyruvic acid using a cell-free extract of *L. lactis* subspecies *cremoris*.
Figure 5 gives the concentration of the metabolites glucose, pyruvic acid, propionic acid and acetic acid in a two-stage anaerobic/aerobic fermentation.

## Claims

1. Process for preparing an aroma product containing a high concentration of α-acetolactic acid by subjecting at least one food product constituent to lactic acid bacterial matter, which comprises subjecting a sufficient amount of said at least one food product constituent to lactic acid bacteria which are no longer in their logarithmic growth phase or to a cell extract or a cell lysate of the lactic acid bacteria.

2. Process according to Claim 1, wherein the lactic acid bacteria have been separated from their culture medium.

3. Process according to Claim 1 or 2, wherein the food product constituent is selected from citric acid, lactic acid and pyruvic acid and mixtures thereof.

4. Process according to Claim 3, wherein the pyruvic acid is obtained from a culture of at least one bacterium of the genus *Propionibacterium*, preferably of the species *Propionibacterium freundenreichii*.

5. Process according to Claim 4, wherein a medium is used which is obtainable by culturing at least one bacterium of the genus *Propionibacterium* in an anaerobic nutrient medium to produce biomass and subsequently continuing to culture aerobically in a medium containing lactic acid until a pyruvate level of at least 3 g/l, preferably at least 5 g/l, and more preferably at least 10 g/l is reached.

6. Process according to any of Claims 1-5, wherein the lactic acid bacteria are selected from the group consisting of the genera *Lactococcus*, *Streptococcus, Lactobacillus*, *Leuconostoc* and *Pediococcus*, preferably of the species *Lactococcus lactis*, more preferably of *Lactococcus lactis* subspecies *lactis*.

7. Process according to Claim 6, wherein a bacterium of *Lactococcus lactis* subspecies *lactis* biovar *diacetilactis*, preferably *Lactococcus lactis* subspecies *lactis* biovar *diacetilactis* strain SD803, is used.

8. Aroma product obtainable by the process according to any of Claims 1-7, containing α-acetolactic acid in addition to other food fermentation products, which comprises at least 0.2 wt.%, preferably at least 0.4 wt.% of α-acetolactic acid.

9. Aroma product according to Claim 8, wherein the product is dry and comprises at least 0.2 wt.%, preferably at least 1 wt.%, more preferably at least 2 wt.% of α-acetolactic acid.

10. Aromatized food material which comprises an aroma product according to Claim 8 or 9, or an aroma product obtained according to a process according to any of Claims 1-7.

11. Process of preparing an aromatized food material according to Claim 10, wherein the aroma product is mixed with a pre-dried food material, for example whey powder, skim milk powder, wheat flour or a polysaccharide.

## Patentansprüche

1. Verfahren zur Herstellung eines Aromaprodukts, das eine hohe Konzentration an α-Acetomilchsäure enthält, indem mindestens ein Nahrungsmittelbestandteil einer Milchsäurebakterienmasse unterworfen wird, welches umfaßt, daß eine hinreichende Menge dieses mindestens einen Nahrungsmittelbestandteils Milchsäurebakterien, welche sich nicht mehr in ihrer logarithmischen Wachstumsphase befinden, oder einem Zellextrakt oder Zell-Lysat der Milchsäurebakterien unterworfen wird.

2. Verfahren nach Anspruch 1, worin die Milchsäurebakterien von ihrem Kulturmedium abgetrennt wurden.

3. Verfahren nach Anspruch 1 oder 2, worin der Nahrungsmittelbestandteil aus Zitronensäure, Milchsäure und Brenztraubensäure und Mischungen davon ausgewählt ist.

4. Verfahren nach Anspruch 3, worin Brenztraubensäure aus einer Kultur von mindestens einem Bakterium der Gattung *Propionibacterium*, vorzugsweise der Spezies *Propionibacterium freudenreichii*, gewonnen ist.

5. Verfahren nach Anspruch 4, worin ein Medium verwendet wird, welches dadurch erhältlich ist, daß man mindestens ein Bakterium der Gattung *Propionibacterium* in einem anaeroben Nährmedium kultiviert, um Biomasse herzustellen, und anschließend die Kultur aerob in einem milchsäurehaltigen Medium fortführt, bis eine Pyruvatkonzentration von mindestens 3 g/l, vorzugsweise mindestens 5 g/l, und besonders bevorzugt von mindestens 10 g/l, erreicht ist.

6. Verfahren nach einem der Ansprüche 1 bis 5, worin die Milchsäurebakterien aus der Gruppe bestehend aus den Gattungen *Lactococcus*, *Streptococcus*, *Lactobacillus*, *Leuconostoc* und *Pediococcus*, vorzugsweise aus den Spezies *Lactococcus lactis*, besonders bevorzugt aus *Lactococcus lactis*, subspecies *lactis* ausgewählt sind.

7. Verfahren nach Anspruch 6, worin ein Bakterium von *Lactococcus lactis* subspecies *lactis* biovar. *diacetilactis*, vorzugsweise *Lactococcus lactis* subspecies *lactis* biovar. *diacetilactis* Stamm SD803, verwendet wird.

8. Aromaprodukt, erhältlich nach einem Verfahren nach einem der Ansprüche 1-7, das neben anderen Nahrungsmittelfermentationsprodukten α-Acetomilchsäure enthält und mindestens 0,2 Gew.-%, vorzugsweise mindestens 0,4 Gew.-%, α-Acetomilchsäure enthält.

9. Aromaprodukt nach Anspruch 8, worin das Produkt trocken ist und mindestens 0,2 Gew.-%, vorzugsweise mindestens 1 Gew.-%, besonders bevorzugt mindestens 2 Gew.-%, α-Acetomilchsäure umfaßt.

10. Aromatisierte Nahrungsmittel, die ein Aromaprodukt nach Anspruch 8 oder 9, oder ein Aromaprodukt, das nach einem Verfahren nach einem der Ansprüche 1-7 erhalten wurde, umfassen.

11. Verfahren zur Herstellung eines aromatisierten Nahrungsmittels nach Anspruch 10, worin das Aromaprodukt mit einem vorgetrockneten Nahrungsmittel, z.B. Molkepulver, Magermilchpulver, Weizenmehl oder einem Polysaccharid, vermischt wird.

## Revendications

1. Procédé pour la préparation d'un produit d'arôme contenant une forte concentration d'acide α-acétolactique en soumettant au moins un ingrédient de produit alimentaire à une matière bactérienne d'acide lactique, et comprenant les étapes consistant à soumettre une quantité suffisante d'au moins un desdits ingrédients de produit alimentaire à des bactéries d'acide lactique ne se trouvant plus dans leur phase de croissance logarithmique ou à un extrait de cellule ou un lysat de cellule des bactéries d'acide lactique.

2. Procédé selon la Revendication 1, dans lequel les bactéries d'acide lactique ont été séparées de leur milieu de culture.

3. Procédé selon la Revendication 1 ou 2, dans lequel l'ingrédient de produit alimentaire est sélectionné parmi de l'acide citrique, de l'acide lactique, de l'acide pyruvique et des mélanges de ceux-ci.

4. Procédé selon la Revendication 3, dans lequel l'acide pyruvique est obtenu à partir d'une culture d'au moins une bactérie de l'espèce *Propionibacterium*, de préférence de l'espèce *Propionibacterium freundenreichii*.

5. Procédé selon la Revendication 4, dans lequel on utilise un milieu pouvant être obtenu en cultivant au moins une bactérie de l'espèce *Propinibacterium* dans un milieu nutritif anaérobie pour produire de la biomasse, et en continuant ensuite la culture de façon anaérobie dans un milieu contenant de l'acide lactique, jusqu'à obtention d'une quantité de pyruvate au moins égale à 3 g/l, de préférence au moins égale à 5 g/l, et de façon plus préférentielle au moins égale à 10 g/l.

6. Procédé selon l'une quelconque des Revendications 1 à 5, dans lequel les bactéries d'acide lactique sont sélectionnées parmi le groupe composé des espèces *Lactococcus*, *Streptococcus*, *Lactobacillus*, *Leuconostoc* et *Pediococcus*, de préférence des espèces *Lactococcus lactis*, de façon plus préférentielle de l'espèce *Lactococcus lactis* du genre *lactis.*

7. Procédé selon la Revendication 6, dans lequel on utilise une bactérie de *Lactococcus lactis* de la sous-espèce *lactis biovar diacetilactis*, de préférence de *Lactococcus lactis* de la sous-espèce *lactis biovar diacetilactis* lignée SD803.

8. Produit d'arôme pouvant être obtenu par le procédé selon l'une quelconque des Revendications 1 à 7 et contenant de l'acide α-acétolactique en plus d'autres produits de fermentation alimentaire, qui comprend au moins 0,2 % en masse, de préférence au moins 0,4 % en masse d'acide α-acétolactique.

9. Produit d'arôme selon la Revendication 8, dans lequel le produit est sec et comprend au moins 0,2% en masse, de préférence au moins 1 % en masse, de façon plus préférentielle au moins 2% en masse, d'acide α-acétolactique.

10. Matière alimentaire aromatisée comprenant un produit d'arôme selon la Revendication 8 ou 9, ou un produit d'arôme obtenu selon un procédé en accord avec l'une quelconque des Revendications 1 à 7.

11. Procédé pour la préparation d'une matière alimentaire aromatisée selon la Revendication 10, dans lequel le produit d'arôme est mélangé avec une matière alimentaire pré-séchée, par exemple de la poudre de petit-lait, de la poudre de lait écrémé, de la farine de froment ou un polysaccharide.
